# EUROPEAN PATENT SPECIFICATION

(11) **EP 2 101 826 B1**
(45) Date of publication and mention of the grant of the patent: **23.06.2010**
(21) Application number: 07860908.8
(22) Date of filing: 11.12.2007
(51) Int. Cl.: A61K 51/08

(54) **RADIOLABELLED PEPTIDE BASED COMPOUNDS AND USES THEREOF**
RADIOAKTIV MARKIERTE VERBINDUNGEN AUF PEPTIDBASIS UND IHRE VERWENDUNG
COMPOSÉS À BASE DE PEPTIDES MARQUÉS RADIOACTIVEMENT ET LEURS UTILISATIONS

(30) Priority: 11.12.2006 US 869382 P
(43) Date of publication of application: 23.09.2009
(73) Proprietor: GE HEALTHCARE AS, 0401 Oslo (NO)
(72) Inventor: ENGELL, Torgrim, 0401 Oslo (NO); FAIRWAY, Steven, 0401 Oslo (NO); HENRIKSEN, Ingrid, 0401 Oslo (NO)
(74) Representative: Canning, Lewis R.
(86) International application number: PCT/NO2007/000434
(87) International publication number: WO 2008/072973

(56) References cited:
- WO-A-03/006491
- WO-A-2004/080492
- INDREVOLL B ET AL: "NC-100717: A versatile RGD peptide scaffold for angiogenesis imaging" BIOORGANIC & MEDICINAL CHEMISTRY LETTERS, PERGAMON, ELSEVIER SCIENCE, GB, vol. 16, no. 24, 26 September 2006 (2006-09-26), pages 6190-6193, XP025106619 ISSN: 0960-894X [retrieved on 2006-12-15]
- GRIERSON J R ET AL: "A RADIOSYNTHESIS OF FLUORINE-18 FLUOROMISONIDAZOLE" JOURNAL OF NUCLEAR MEDICINE, SOCIETY OF NUCLEAR MEDICINE, RESTON, VA, US, vol. 30, no. 3, 1 March 1989 (1989-03-01), pages 343-350, XP000033832 ISSN: 0161-5505
- COENEN H H: "Fluorine-18 labeling methods: Features and possibilities of basic reactions" ERNST SCHERING RESEARCH FOUNDATION WORKSHOP, SPRINGER, BERLIN, DE, vol. 62, 7 December 2005 (2005-12-07), pages 15-50, XP008097616 ISSN: 0947-6075

## Description

### Field of the Invention

The present invention relates to radiolabelled peptide-based compounds and their use for diagnostic imaging using positron emission tomography (PET). Such compounds may thus be used for diagnosis or therapy of, for example, malignant diseases, heart diseases, endometriosis, inflammation-related diseases, rheumatoid arthritis and Kaposi's sarcoma.

### Background of the Invention

The application of radiolabelled bioactive peptides for diagnostic imaging is gaining importance in nuclear medicine. Biologically active molecules which selectively interact with specific cell types are useful for the delivery of radioactivity to target tissues. For example, radiolabelled peptides have significant potential for the delivery of radionuclides to tumours, infarcts, and infected tissues for diagnostic imaging and radiotherapy. ¹⁸F, with its half-life of approximately 110 minutes, is the positron-emitting nuclide of choice for many receptor imaging studies. Therefore, ¹⁸F-labelled bioactive peptides have great clinical potential because of their utility in PET to quantitatively detect and characterise a wide variety of diseases.

New blood vessels can be formed by two different mechanisms: vasculogenesis or angiogenesis. Angiogenesis is the formation of new blood vessels by branching from existing vessels. The primary stimulus for this process may be inadequate supply of nutrients and oxygen (hypoxia) to cells in a tissue. The cells may respond by secreting angiogenic factors, of which there are many; one example, which is frequently referred to, is vascular endothelial growth factor (VEGF). These factors initiate the secretion of proteolytic enzymes that break down the proteins of the basement membrane, as well as inhibitors that limit the action of these potentially harmful enzymes. The other prominent effect of angiogenic factors is to cause endothelial cells to migrate and divide. Endothelial cells that are attached to the basement membrane, which forms a continuous sheet around blood vessels on the contralumenal side, do not undergo mitosis. The combined effect of loss of attachment and signals from the receptors for angiogenic factors is to cause the endothelial cells to move, multiply, and rearrange themselves, and finally to synthesise a basement membrane around the new vessels.

Angiogenesis is prominent in the growth and remodelling of tissues, including wound healing and inflammatory processes. Tumours must initiate angiogenesis when they reach millimetre size in order to keep up their rate of growth. Angiogenesis is accompanied by characteristic changes in endothelial cells and their environment. The surface of these cells is remodelled in preparation for migration, and cryptic structures are exposed where the basement membrane is degraded, in addition to the variety of proteins which are involved in effecting and controlling proteolysis. In the case of tumours, the resulting network of blood vessels is usually disorganised, with the formation of sharp kinks and also arteriovenous shunts. Inhibition of angiogenesis is also considered to be a promising strategy for antitumour therapy. The transformations accompanying angiogenesis are also very promising for diagnosis, one example being malignant disease, but the concept also shows great promise in inflammation and a variety of inflammation-related diseases, including atherosclerosis, the macrophages of early atherosclerotic lesions being potential sources of angiogenic factors.

WO 2003/006491 describes peptide-based compounds which target integrin receptors associated with angiogenesis. International application PCT/GB2004/001052 describes methods suitable for labelling biologically active vectors with ¹⁸F. However, there exists a need for further peptide-based compounds having utility for diagnostic imaging techniques such as PET.

### Detailed Description of the Invention

The present invention relates to peptide-based compounds and their use for diagnostic imaging using PET. The ¹⁸F peptide based compounds discussed herein show a biodistribution (less binding in liver and other organs) that is about 15% higher in comparison to any relatively successful prior agents developed.

One embodiment of the present invention encompasses a compound of formula (1), 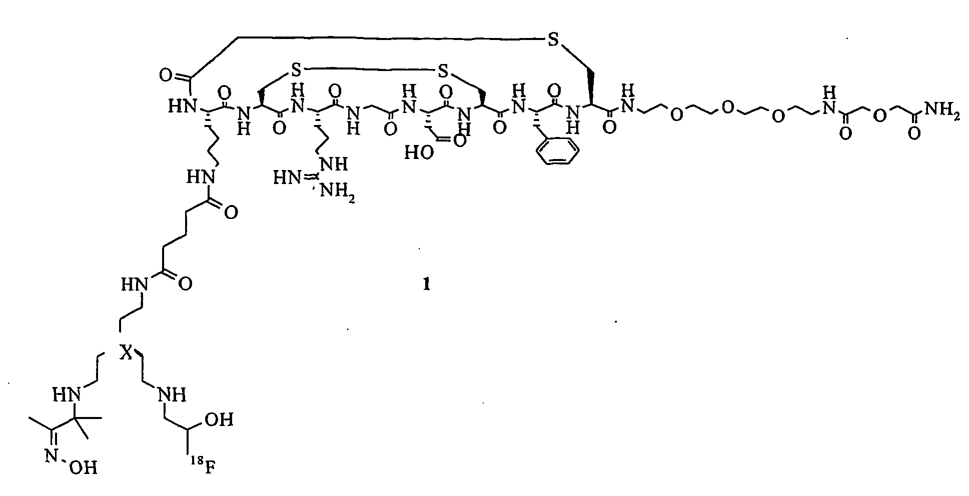 wherein X is a carbon or nitrogen.

Yet another embodiment of the invention entailes a compound of formula 2, 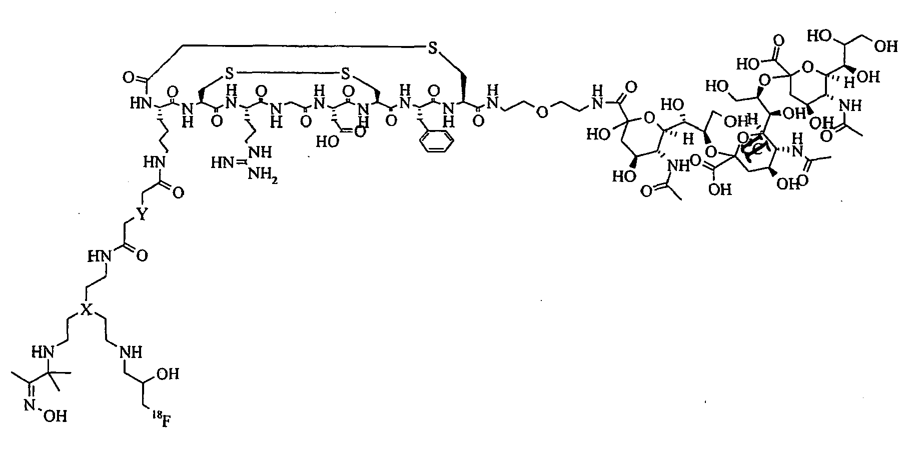 wherein Y is a carbon or oxygen and X is a nitrogen or carbon.

PET imaging agents. Compounds of formula (1) and (2) are prepared by standard methods of peptide synthesis, for example, solid-phase peptide synthesis, as described in Atherton, E. and Sheppard, R.C.; "Solid Phase Synthesis"; IRL Press: Oxford, 1989. Incorporation of the aminoxy group in a compound of formula (I) is achieved by formation of a stable amide bond formed by reaction of a peptide amine function with an activated acid and introduced either during or following the peptide synthesis.

Still a further embodiment of the invention depicts a radiopharmaceutical composition comprising an effective amount of a compound of formula 1 or 2, together with one or more pharmaceutically acceptable adjuvants, excipients or diluents.

Yet another embodiment of the present invention depicts a compound of formula 1 or 2 for medical use particularly in the in vivo diagnosis or imaging, for example by PET, of a disease or condition associated with angiogenesis.

The term "diseases and conditions associated with angiogenesis" includes those diseases and conditions referred to below. Reference is also made in this regard to WO 98/47541.

Diseases and conditions associated with angiogenesis include different forms of cancer and metastasis, for example, breast, skin, colorectal, pancreatic, prostate, lung or ovarian cancer.

Other diseases and conditions associated with angiogenesis are inflammation (for example, chronic inflammation), atherosclerosis, rheumatoid arthritis and gingivitis.

Further diseases and conditions associated with angiogenesis are arteriovenous alformations, astrocytomas, choriocarcinomas, glioblastomas, gliomas, hemangiomas (childhood, capillary), hepatomas, hyperplastic endometrium, ischemic myocardium, endometriosis, Kaposi sarcoma, macular degeneration, melanoma, neuroblastomas, occluding peripheral artery disease, osteoarthritis, psoriasis, retinopathy (diabetic, proliferative), scleroderma, seminomas and ulcerative colitis.

Still another embodiment of the present invention depicts a use of a compound of formula 1 or 2 for the manufacture of a radiopharmaceutical for use in the method of *in vivo* imaging.

Yet another embodiment of the present invention shows a method of generating an image of a human or animal body comprising administering a compound of claim 1 or 2 to said body and generating an image of at least a part of said body to which said compound has distributed using PET.

Another embodiment of the present invention shows a method of monitoring the effect of treatment of a human or animal body with a drug to combat a condition associated with cancer, preferably angiogenesis, said method comprising administering to said body a compound of formula 1 or 2 and detecting the uptake of said compound by cell receptors said administration and detection optionally but preferably being effected before, during and after treatment with said drug.

### Examples

The invention is further described in the following example.

The following abbreviations are used.
- NMR:: nuclear magnetic resonance
- TFA:: trifluoroacetic acid
- Boc:: t-butoxycarbonyl

A precursor for the novel ¹⁸F peptide based compounds are made from a reaction between A (available from Bachem) and a by-product from the synthesis of B (mono-alkylated by-product). See Scheme 1 below.

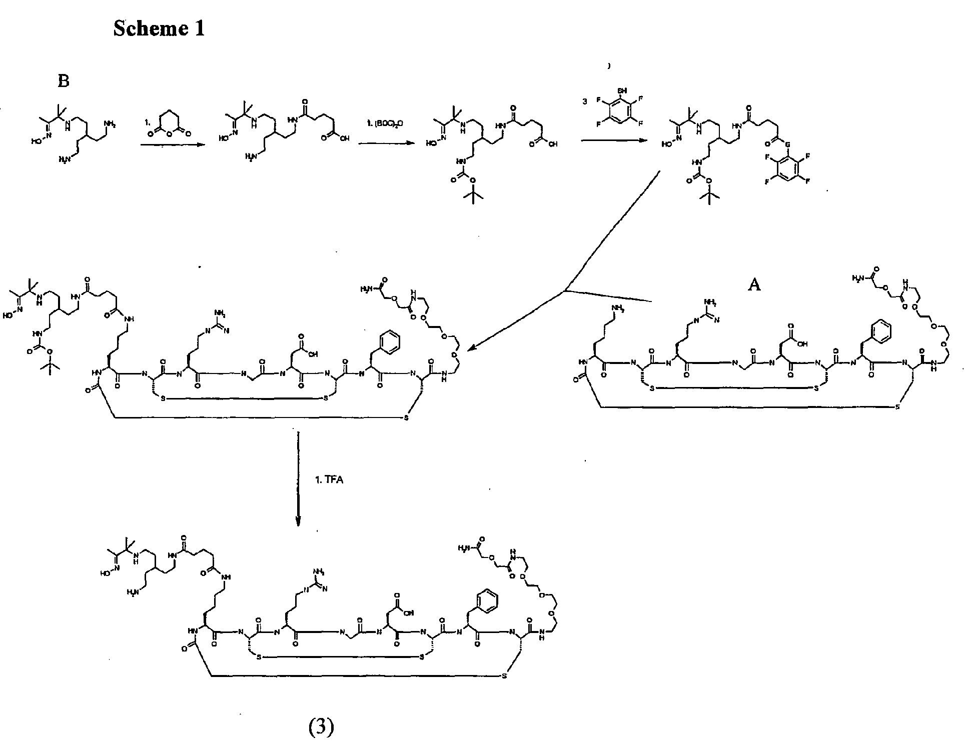

The novel ¹⁸F peptide based compound disclosed herein (4) wherein Z is carbon is made by reacting the precursor (3) shown above with ¹⁸F epifluorohydrin or with 1-fluoro-3-chloro-propan-2-ol. See Scheme 2 below.

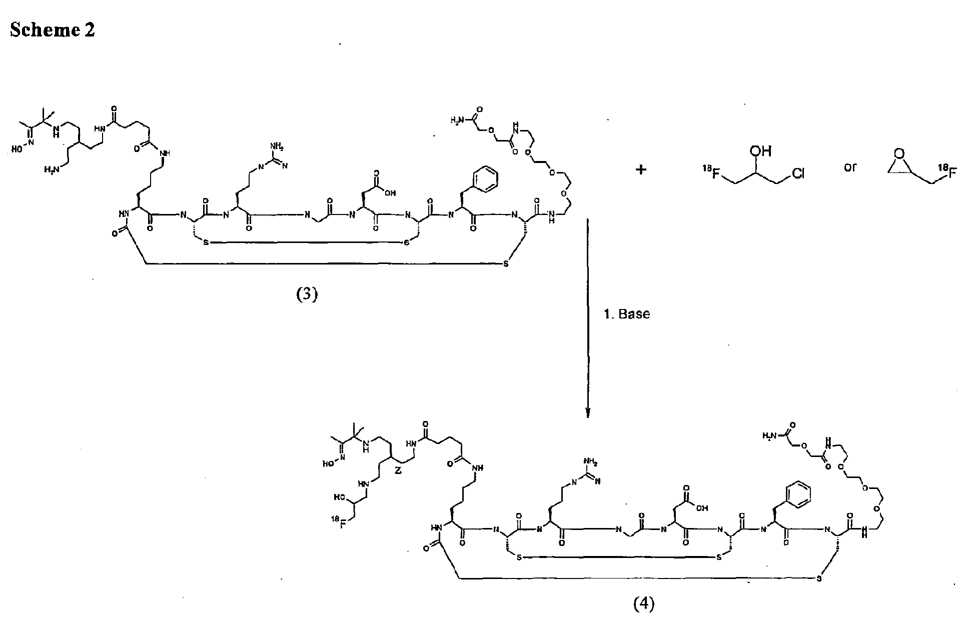

The fact that starting materials are readily available and known can aid in reducing development time and costs. The present invention also suggests that the novel angiogenesis ¹⁸F peptide based compounds have a superior biological profile/properties compared to other compounds. The novel ¹⁸F peptide based compounds discussed herein show a biodistribution (less binding in liver and other organs) that is about 15% higher in comparison to any relatively successful prior agents developed.

The formation of the 3-chloro-2-hydroxy-1-¹⁸fluoride at room temperature has been proved by both¹⁹F- and and a 400 MegaHertz ¹H-NMR spectrometer. The laboratory tests using the epoxide, as depicted above in scheme 2, to introduce ¹⁸F fluoride to an organic molecule was performed in water at room temperature and yielded a 30% relative amount of the organic fluoride target molecule. This amount obtained is about 2-3 times greater than the recovery amount of the organic fluoride target molecule compared to ¹⁸F-labeling using tosyl (or a similar leaving group such as triflate or mesylate) as a leaving group.

## Claims

1. A compound of formula (1) which is 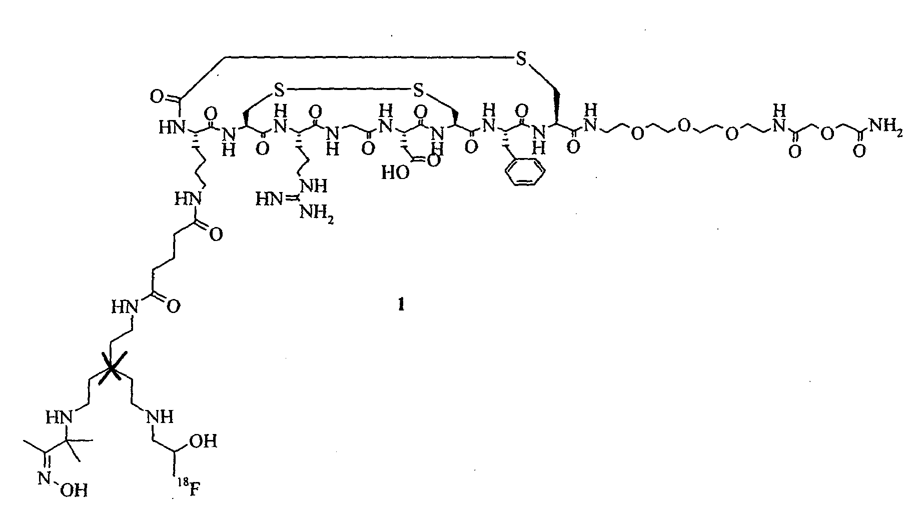 wherein X is a carbon or a nitrogen.

2. A compound of formula (2) 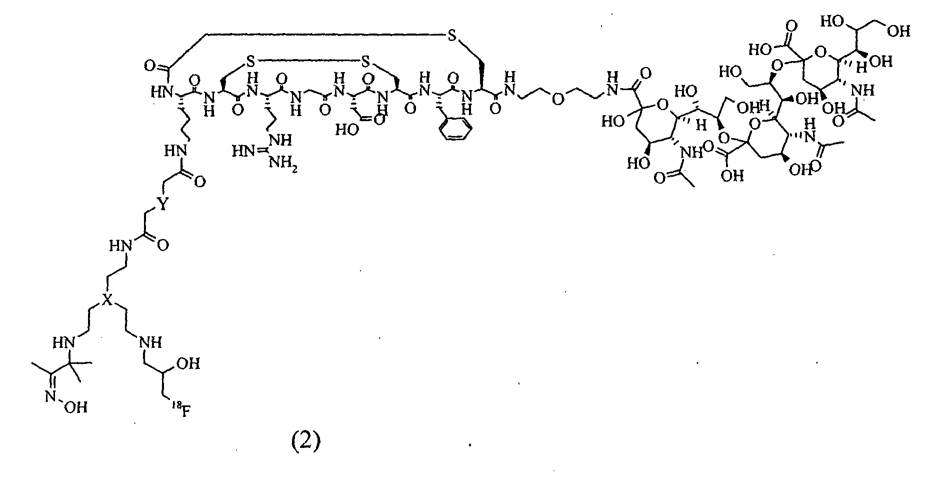 wherein Y is carbon or oxygen and X is nitrogen or carbon.

3. A radiopharmaceutical composition comprising an effective amount of a compound of claim 1 or 2, together with one or more pharmaceutically acceptable adjuvants, excipients or diluents.

4. A compound of claim 1 or 2 for medical use particularly in the in vivo diagnosis or imaging, for example by PET, of a disease or condition associated with angiogenesis.

5. Use of a compound of claim 1 or 2 for the manufacture of a radiopharmaceutical for use in the method of *in vivo* imaging.

6. A method of generating an image of a human or animal body comprising administering a compound of claim 1 or 2 to said body and generating an image of at least a part of said body to which said compound has distributed using PET.

7. A method of monitoring the effect of treatment of a human or animal body with a drug to combat a condition associated with cancer, preferably angiogenesis, said method comprising administering to said body a compound of claim 1 or 2 and detecting the uptake of said compound by cell receptors said administration and detection optionally but preferably being effected before, during and after treatment with said drug.

## Patentansprüche

1. Verbindung der Formel (1), die lautet 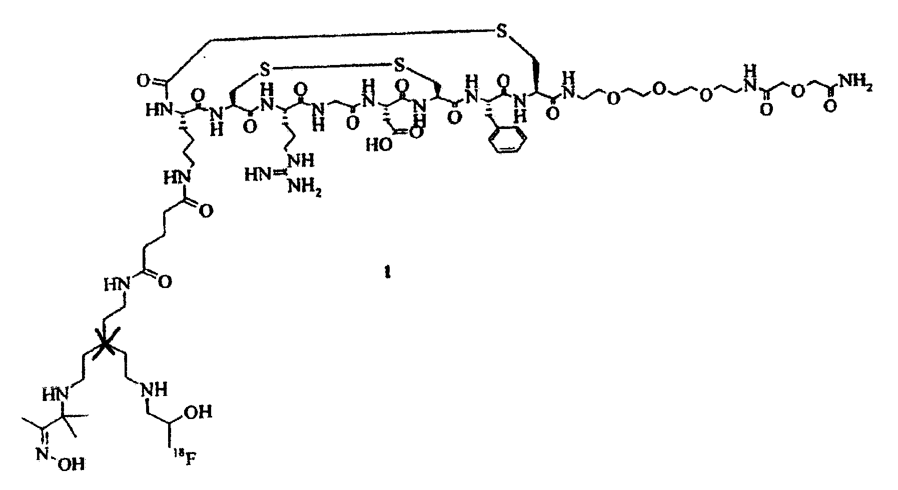 wobei X für Kohlenstoff oder Stickstoff steht.

2. Verbindung der Formel (2) 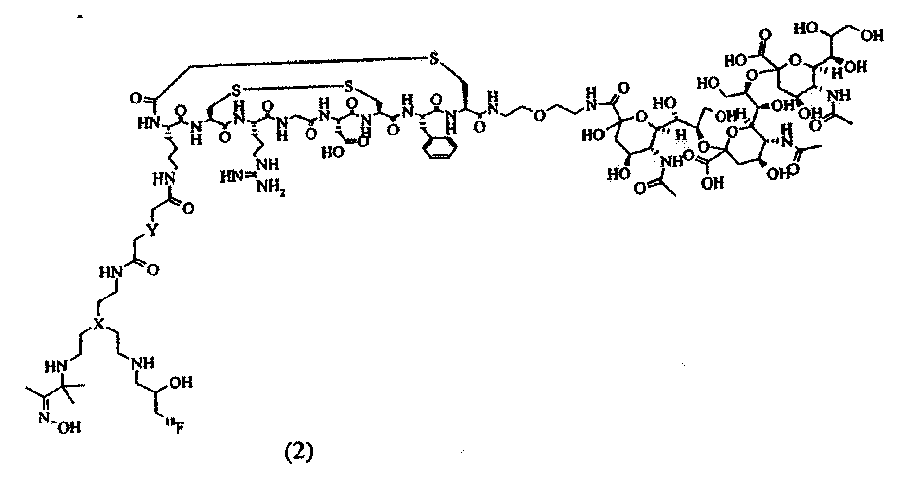 wobei Y für Kohlenstoff oder Sauerstoff steht und X für Stickstoff oder Kohlenstoff steht.

3. Radiopharmazeutische Zusammensetzung, umfassend eine wirksame Menge einer Verbindung des Anspruchs 1 oder 2, zusammen mit einem oder mehreren pharmazeutisch annehmbaren Hilfsstoffen, Trägerstoffen oder Verdünnungsmitteln.

4. Verbindung nach Anspruch 1 oder 2 zur medizinischen Verwendung insbesondere in der *in vivo* Diagnose oder Bildgebung, beispielsweise mittels PET, von einer Erkrankung oder einem Leiden, die mit Angiogenese assoziiert sind.

5. Verwendung einer Verbindung nach Anspruch 1 oder 2 für die Herstellung eines Radiopharmakons zum Einsatz im Verfahren der *in vivo* Bildgebung.

6. Verfahren zur Erzeugung eines Bildes von einem Menschen- oder Tierkörper, umfassend das Verabreichen einer Verbindung des Anspruchs 1 oder 2 an den Körper und das Erzeugen eines Bildes von zumindest einem Teil des Körpers, an der die Verbindung verabreicht wurde, mittels PET.

7. Verfahren zur Überwachung der Wirksamkeit einer Behandlung eines Menschen- oder Tierkörpers mit einem Arzneimittel, um einen mit Krebs assoziierten Zustand, bevorzugt Angiogenese, zu bekämpfen, wobei das Verfahren das Verabreichen einer Verbindung des Anspruchs 1 oder 2 an den Körper und das Nachweisen der Aufnahme der Verbindung durch Zellrezeptoren umfasst, wobei Verabreichung und Nachweis optional, aber bevorzugt vor, während und nach Behandlung mit dem Arzneimittel durchgeführt werden.

## Revendications

1. Composé selon la formule (1) qui est 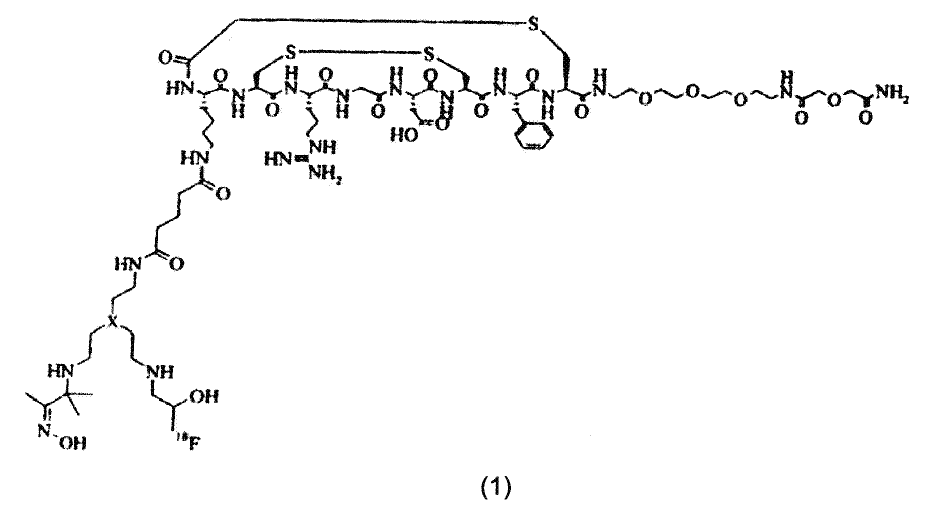 dans laquelle X représente un carbone ou un azote.

2. Composé selon la formule (2) 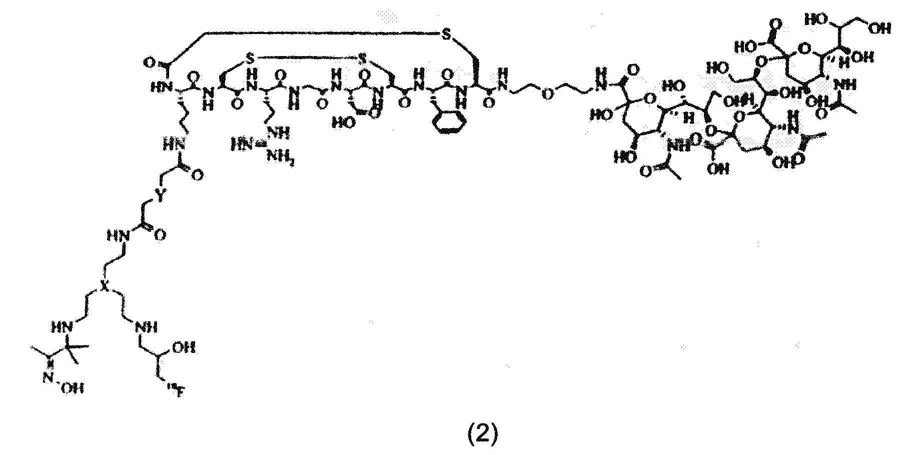 dans laquelle Y représente un carbone ou un oxygène et X représente un azote ou un carbone.

3. Composition radio-pharmaceutique comportant une quantité efficace d'un composé selon la revendication 1 ou 2, ainsi qu'un ou plusieurs adjuvants, excipients ou diluants pharmaceutiquement acceptables.

4. Composé selon la revendication 1 ou 2 destiné à une utilisation médicale, en particulier, dans le diagnostic ou l'imagerie in vivo, par exemple, par tomographie PET, d'une maladie ou une condition associée à l'angiogénèse.

5. Utilisation d'un composé selon la revendication 1 ou 2 afin d'assurer la fabrication d'un agent radio-pharmaceutique en vue d'une utilisation dans le procédé d'imagerie in vivo.

6. Procédé de production d'une image d'un corps humain ou animal comprenant l'administration d'un composé selon la revendication 1 ou 2 dans ledit corps et la production d'une image d'au moins une partie dudit corps dans lequel ledit composé a été administré en utilisant la tomographie PET.

7. Procédé de surveillance de l'effet du traitement d'un corps humain ou animal avec un médicament destiné à combattre une condition associée au cancer, de préférence, à l'angiogénèse, ledit procédé comprenant l'administration dans ledit corps d'un composé selon la revendication 1 ou 2 et la détection de l'assimilation dudit composé par des récepteurs cellulaires, lesdites administration et détection étant, en variante, mais de préférence, réalisées avant, pendant et après le traitement avec ledit médicament.
